# EUROPEAN PATENT SPECIFICATION

(11) **EP 1 704 843 B1**
(45) Date of publication and mention of the grant of the patent: **30.11.2016**
(21) Application number: 04807236.7
(22) Date of filing: 17.12.2004
(51) Int. Cl.: A61B 50/30

(54) **PACKING MATERIAL AND MEDICAL INSTRUMENT SET PACKAGE**
VERPACKUNGSMATERIAL UND VERPACKUNG FÜR EIN MEDIZINISCHES INSTRUMENTENSET
MATERIAU DE CONDITIONNEMENT ET CONDITIONNEMENT D'ENSEMBLE D'INSTRUMENTS MEDICAUX

(30) Priority: 16.01.2004 JP 2004009365
(43) Date of publication of application: 27.09.2006
(73) Proprietor: Terumo Kabushiki Kaisha, Tokyo 151-0072 (JP)
(72) Inventor: IMAI, Tadashi c/o Terumo Kabushiki Kaisha, Ashigarakami-gun, Kanagawa 2590151 (JP)
(74) Representative: TBK
(86) International application number: PCT/JP2004/018878
(87) International publication number: WO 2005/067863

(56) References cited:
- EP-A1- 1 293 222
- WO-A2-02/058564
- CA-A1- 1 215 658
- JP-A- 2001 070 443
- JP-A- 2001 070 443
- JP-A- 2003 002 364
- JP-A- 2003 002 364
- JP-U- 3 000 208
- JP-U- 61 166 976
- US-A- 5 022 521
- US-A1- 2004 004 019

## Description

### TECHNICAL FIELD

The present invention relates to a packaging material to house a medical instrument and also to a package for a medical instrument set, including a packaging material having a medical instrument set housed therein.

### BACKGROUND ART

Among known blood processing devices is a blood bag system which centrifugally separates whole blood (collected from donors) into three kinds of blood products for transfusion: concentrated red blood cells (CRC), platelet concentrated (PC), and platelet-poor plasma (PPP).

The previously stored blood products for transfusion are usually processed to remove leukocytes immediately before transfusion into patients, in cases where it is necessary to avoid possible side effects induced by leukocytes present in the blood products.

It is known that blood products for transfusion have good quality if whole blood, which is collected by blood donation, has leukocytes removed before separation and storage. In order to remove leukocytes before blood separation and storage there has been developed a leukocyte removing filter system (in-line filter) including a blood bag system and a filter, which are integrally connected together. (See Patent Document 1 below.)

The above-mentioned system is composed of a blood collecting needle, a filter, and a plurality of bags (blood collecting bag and blood component bag), which are connected together by tubes to form a blood processing circuit.

The blood processing circuit is usually housed in a packaging material in a sterilized state, wherein the blood processing circuit is taken out of the package (with its cover broken and opened) at the time of use.

The disadvantage of taking the entire blood processing circuit out of the package is that the blood collecting needle (which is initially required for blood collection) has to be picked out of the entire blood processing circuit, and the procedure for blood collection is hampered by the filter and blood component bags (which are not necessary for blood collection but become necessary in later steps). Therefore, the blood processing circuit is inconvenient to handle.
Patent Document 1
   Japanese Patent Publication No. 6-59304

In document JP 2001 070443 A, which forms the basis of the preamble of claim 1, a medical instrument set is housed in a bag. A plurality of such bags is contained in an outer packaging in the form of a box and a lid is provided to the box in order to close the box.

In document JP 2003 002364 A, a food package is disclosed. The food is contained in a cup or the like having a lid for sealing the packaging. Further, on the lower side of the lid, certain compartments are provided for containing e.g. spicery or herbage separately from the rest of the food. The compartments are designed such that before opening the lid of the cup, the compartments are opened, so that the spicery or herbage drops on the food to be mixed therewith. In order to open the compartments, a buttstrap is provided to be pulled before removal of the lid of the cup.

### DISCLOSURE OF THE INVENTION

Claim 1 defines the scope of the invention and the dependant claims disclose the preferred embodiments.

It is an object of the present invention to provide a packaging material and a package for a medical instrument set, which are designed for easy use, and so that medical instruments can be taken out of the package sequentially.

The above-mentioned object of the present invention is achieved by providing a packaging material according to claim 1.

The packaging material according to the present invention permits the operator to take only a necessary medical instrument out of the medical instrument set, which is composed of a plurality of medical instruments, with the remainder of the medical instruments being left in he package, in a previously established order, such that the medical instrument (or part thereof) to be used first appears as soon as the lid of the package is opened. Therefore, the packaging material is convenient for easy, rapid, and accurate handling of a particular medical instrument selected from the medical instrument set.

The packaging material according to the present invention should preferably have means for expanding the opening.

Such a means expands the opening when necessary after the opening has been unsealed, thereby allowing even large medical instruments to be taken out easily and surely.

The means for expanding the opening should preferably be formed by perforation or thinning at the periphery of the opening so that it can be broken easily.

The perforated or thinned part permits easy and sure breaking.

The means for expanding the opening should preferably be covered by a covering lid.

The covering lid prevents the packaging material 15 from breathing through the perforations 18.

The medical instrument set should preferably be composed of a plurality of medical instruments connected together by tubes.

The medical instrument set, which is housed in the packaging material of the present invention, produces the aforementioned effects more significantly.

The medical instrument set includes at least one bag and one puncture needle, wherein the medical instrument to be used first should preferably be the needle.

The present invention produces the aforementioned effects more significantly when it is applied to the medical instrument set.

The aforementioned medical instrument set should preferably be composed of medical instruments including a first bag for storing collected blood, a blood collecting needle connected to the first bag through a tube, a second bag for storing blood or blood components, and a filter which has an inlet, an outlet, and a filter medium for removing a specific component from the blood introduced through the inlet. The blood collecting needle is the medical instrument that is to be used first.

The medical instrument set according to the present invention facilitates blood collection requiring prompt and adequate operations, in that it permits an operator to take out the blood collecting needle (which is to be used first) from the packaging material, and to stick the needle into a blood vessel quickly, easily and accurately.

The medical instruments in the medical instrument set should preferably be arranged such that the medical instrument (or part thereof) to be used first is bonded to the inside of the lid.

Thus, the medical instrument (or part thereof) to be used first can be easily attached to and detached from the inside of the lid.

The medical instrument (or part thereof) to be used first should preferably be held on the inside of the lid by means of a holding member attached to the inside of the lid. The holding member should preferably be a deformable string-like object.

Thus the medical instrument (or part thereof) to be used first can easily be attached to or detached from the inside of the lid. The deformable string-like holding member easily and securely holds and fixes the medical instrument to be used first, while also facilitating detachment thereof.

The lid should preferably be constructed such that an inside portion thereof includes a pocket, wherein the medical instrument (or part thereof) to be used first is inserted into the pocket.

Thus, a structure is provided, which is suitable for mass production, wherein the medical instrument (or part thereof) to be used first can easily be inserted and taken out.

The lid should preferably have a sheet-like magnet provided on at least a portion thereof, so that the medical instrument (or part thereof) to be used first can be held thereby magnetically.

The structure facilitates attachment and detachment of the medical instrument (or part thereof) to be used first from the inside of the lid.

To achieve the aforementioned objects, the present invention is also directed to a package for a medical instrument set, which includes any of the packaging materials defined in Claims 1 to 14, wherein the medical instrument set is housed inside the packaging material.

The package for a medical instrument set according to the present invention offers the following advantages. An operator is allowed to take out only those necessary medical instruments from the medical instrument set package, which is composed of a plurality of medical instruments, while leaving the other medical instruments inside the package. The package also permits an operator to take out medical instruments from the package in a previously established order. In particular, the package enables an operator to take out a medical instrument (or part thereof), which is to be used first, as soon as the lid is unsealed. This leads to easy, quick and accurate selection of medical instruments necessary for performing operations.

### BRIEF DESCRIPTION OF THE DRAWINGS

FIG. 1 is a perspective view showing an embodiment of the packaging material and the package for a medical instrument set according to the present invention.
FIG. 2 is a perspective view showing the packaging material, from which a part (blood collecting part) of the medical instrument set has been taken out.
FIG. 3 is a plan view showing an example of a medical instrument housed inside the packaging material.
FIG. 4 is a perspective view showing an example of the structure for holding or fixing the medical instrument (or part thereof) that is used first on the inside of the lid.
FIG. 5 is a perspective view showing another example of the structure for holding or fixing the medical instrument (or part thereof) that is used first on the inside of the lid.

### BEST MODE FOR CARRYING OUT THE INVENTION

A detailed description is given below, with reference to the accompanying drawings, of the packaging material and the package for a medical instrument set, in accordance with the present invention.

Embodiments of the packaging material and the package for a medical instrument set according to the present invention shall be described below with reference to the accompanying drawings.

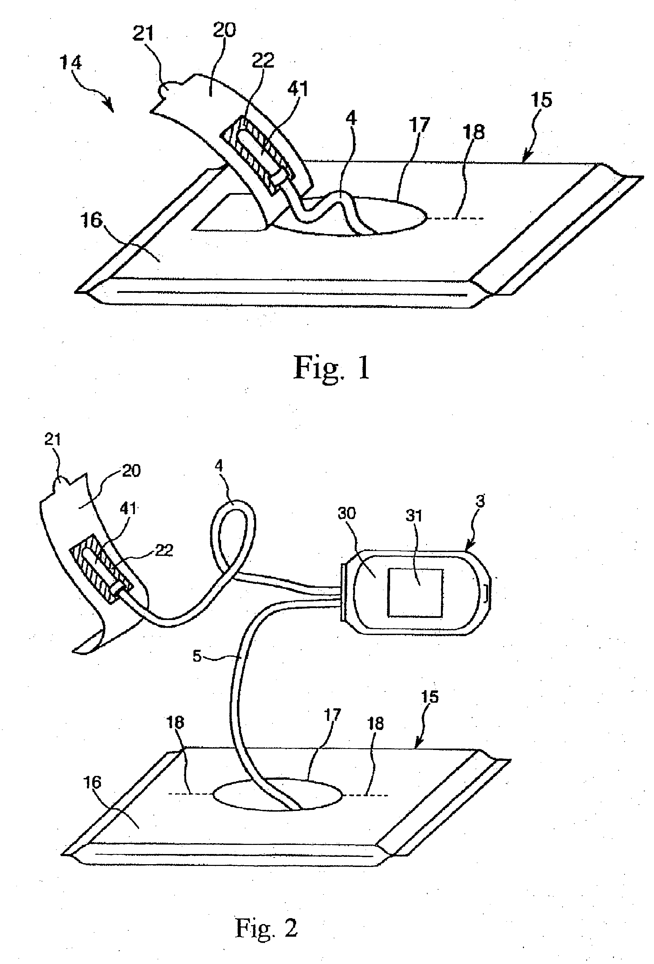

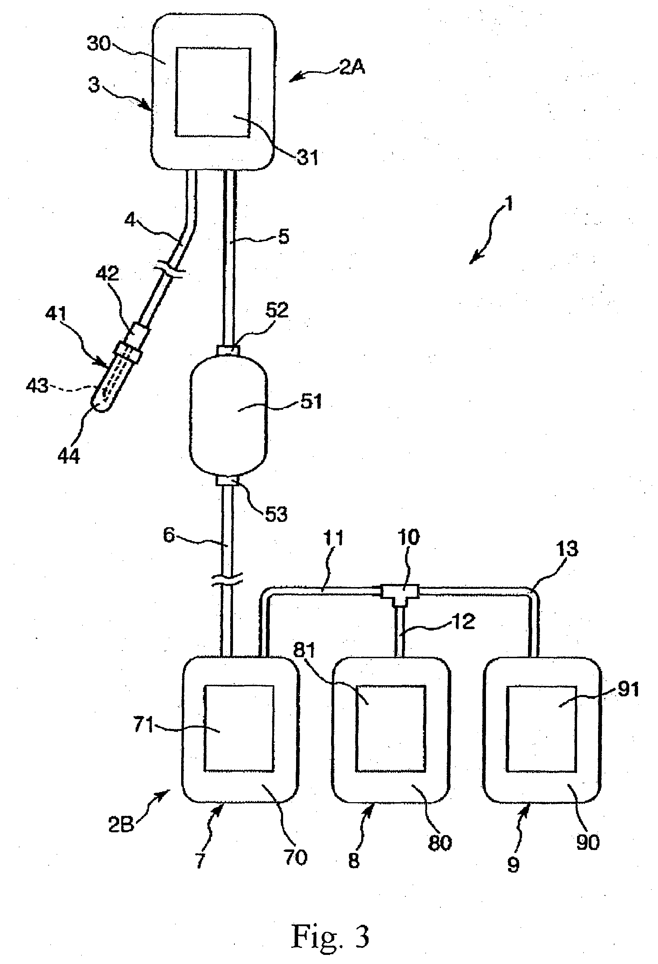

FIG. 1 is a perspective view showing an embodiment of the packaging material and the package for a medical instrument set according to the present invention. FIG. 2 is a perspective view showing the packaging material, from which a part (blood collecting part) of the medical instrument set has been taken out. FIG. 3 is a plan view showing an example of a medical instrument housed inside the packaging material.

First, as shown in FIG. 3, an explanation shall be given concerning the medical instrument set housed inside the packaging material. The medical instrument set shown in FIG. 3 is a blood-processing tool (blood-processing circuit) 1, which is composed of a blood collecting unit 2A and a blood processing unit 2B.

The blood collecting unit 2A is composed of a first bag 3 (blood collecting bag) for storing collected blood, a tube 4 (blood collecting tube) for introducing blood to the first bag 3, a tube 5 (a first tube) for discharging blood from the first bag 3, and a blood collecting needle 41 attached to the end of the tube 4.

The first bag 3 includes a bag body 30, which is formed from laminated flexible sheets that are heat-sealed along the periphery thereof. Incidentally, the bag body 30 of the first bag 3 may take on any shape, such as a cylindrical sheet with both ends thereof being sealed, or a folded flat sheet with three sides thereof being heat-sealed.

The bag body 30 may be made of a polymeric material, such as soft polyvinyl chloride (in the form of a homopolymer or copolymer, a polymer blend, or a polymer alloy incorporated with small amounts of other polymeric materials) or an ethylene-vinyl acetate copolymer.

It is desirable for the first bag 3 to be provided with and contain beforehand an anticoagulant (usually in the form of a liquid), which includes an ACD-A solution, a CPD solution, a CPDA-1 solution, and a heparin sodium solution. The amount of anticoagulant provided inside the bag body 30 should be determined in accordance with the amount of blood to be collected therein.

As shown in FIG. 3, the first bag 3 includes flexible tubes 4 and 5 connected to a lower end thereof. The tubes communicate with the first bag 3 that stores collected blood.

The tube 4 introduces blood into the first bag 3. The tube 5 discharges blood from the first bag 3, and transfers the discharged blood to a filter 51 (discussed later).

A blood collecting needle 41 is attached to one end of the tube 4, which includes a hub 42, a needle tube 43, and a cap 44 mounted on the hub 42 in such a way as to enclose the needle tube 43. The needle tube 43 is made of metal such as tungsten steel, whereas the hub 42 and cap 44 are formed of a resin material.

The tube 5 discharges collected blood from the first bag 3 and transfers the discharged blood to the filter 51 (discussed later). It is desirable for the tube 5 to include, near the first bag 3, a device (not shown) which closes the tube 5 before it is broken and which opens the tube 5 after it has been broken.

The blood processing unit 2B functions to separate the collected blood into several blood components and recover desired components into respective bags. The blood processing unit 2B includes a filter 51 (for filtering out specific components from the blood) and second bags 7, 8, and 9 (for storing blood components), which are connected together by tubes.

In this embodiment, the second bag 7 functions as an erythrocyte bag that eventually stores concentrated red blood cells (CRC), the second bag 8 functions as a platelet bag that eventually stores platelet concentrated (PC), and the second bag 9 functions as a plasma bag that eventually stores platelet-poor plasma (PPP).

The second bags 7, 8, and 9 respectively are defined by bag bodies 70, 80, and 90, each of which is made of flexible laminated sheets that are heat-sealed along the periphery thereof. As in the case of the bag body 30 discussed above, the bag bodies may take on any form, e.g., a cylindrical sheet with both ends thereof being sealed, and a folded flat sheet with three sides thereof being sealed.

The bag bodies 70, 80, and 90 may be made of a polymeric material, such as soft polyvinyl chloride (in the form of a homopolymer or a copolymer, a polymer blend, or a polymer alloy incorporated with small amounts of other polymeric materials) or an ethylene-vinyl acetate copolymer.

It is desirable for the second bag 9 (or the bag body 90) to be provided with and contain beforehand therein an erythrocyte preserver (usually in the form of a liquid), which includes a SAGM solution, an OPTISOL solution, and a MAP solution. The amount of erythrocyte preserver provided inside the bag body 90 should be determined in accordance with the amount of blood to be collected.

As shown in FIG. 3, the second bag 7 includes flexible tubes 6 and 11 connected to an upper end thereof. The tube 6 communicates with the blood component storage space inside the second bag 7, so that the tube 6 introduces blood which has passed through the filter 51 into the second bag 7. The tube 11 transfers the blood component from the second bag 7 to the second bag 8.

The second bags 8 and 9 include respective flexible tubes 12 and 13, connected to upper sides thereof, as shown in FIG. 3. The tube 12 communicates with the blood component storage space inside the second bag 8, whereas the tube 13 communicates with the blood component storage space inside the second bag 9. Each of the ends of the tubes 11, 12, and 13 is connected respectively to three ports of a branching connector 10 (such as a T-tube, a Y-tube, or a three-way cock).

As a result of the aforementioned structure, the second bags 7, 8, and 9 are connected in communication with one another through the tubes 11, 12, and 13 and the branching connector 10.

As shown in FIG. 3, the filter 51 includes an inlet 52 and an outlet 53, to which ends of the tubes 5 and 6 are connected respectively.

The filter 51 may include a bypass tube (not shown), the ends of which are connected to a midway portion of the tubes 5 and 6. In addition, the inlet 52 may include an air vent.

The filter 51 is composed of a housing having a filter medium placed therein. The filter 51 receives blood from the inlet 52 and discharges desired components (or unnecessary components) from the outlet 53 after filtration by the filter medium.

The housing of the filter 51 may be made of any material, such as polycarbonate and soft polyvinyl chloride.

The filter medium in the filter 51 may be made of a porous material or a won-woven fabric of polyether polyurethane, polyester polyurethane, polyethylene terephthalate, or polybutylene terephthalate.

No particular restriction is imposed on the type of filter 51, and the filter 51 may be selected according to its intended use. The filters 51 may be classified as follows, according to the components to be filtered out: leukocyte removing filter, microaggregate removing filter, virus removing filter, and endotoxin removing filter. Other filters, including those which are designed to remove microbes, prions, pathogenic substances, etc., may also be used.

The leukocyte removing filter may be classified into two types, one of which is designed to separate leukocytes only (or to separate one or more than one kind of lymph cells, granular leukocytes, and monocytes), and the other of which is designed to separate leukocytes and platelets. The leukocyte removing filter may also be designed to separate microaggregates together with leukocytes.

The microaggregate removing filter may be classified into two types, one of which is designed to remove microaggregates only, and the other of which is designed to remove microaggregates and platelets.

The virus removing filter may be classified into two types, one of which is designed to remove viruses only (such as HAV, HBV, HCV, HIV, HTLV-1, CMV, parvovirus B19, filovirus, and hantavirus), and the other of which is designed to remove viruses together with either or both of endotoxins and microaggregates. The virus removing filter may also be designed to selectively remove leukocytes and platelets.

The endotoxin removing filter may be classified into two types, one of which is designed to remove endotoxins only, and the other of which is designed to remove either or both of viruses and microaggregates. The endotoxin removing filter may also be designed to selectively remove leukocytes and platelets.

Incidentally, in the following description, the filter 51 is typified by a leukocyte removing filter that separates (filters out) leukocytes from blood.

The tubes 4, 5, 6, 11, 12, and 13 may be made of a polymeric material, such as soft polyvinyl chloride (in the form of a homopolymer or a copolymer, a polymer blend, or a polymer alloy incorporated with small amounts of other polymeric materials) or an ethylene-vinyl acetate copolymer.

The bag bodies 30, 70, 80, and 90 have respective labels 31, 71, 81, and 91 attached thereto by an adhesive. The labels 31, 71, 81, and 91 have adhesive layers on the reverse sides thereof, wherein the labels are attached to surfaces of the respective bag bodies 30, 70, and 90 by the adhesive layers.

The labels 31, 71, 81, and 91 show printed information about the content of the bags carrying them. The information includes the type of blood components, the volume of the bag, the blood group, data concerning blood collection, and details of the donor (such as name, age, and sex). Such information may be represented by characters, numerals, or symbols, or by coding with a bar code or by a two-dimensional code.

The labels 31, 71, 81, and 91 should be firmly stuck to the bag bodies 30, 70, 80, and 90, respectively, and should be tamper-proof.

Because the bag contains an anticoagulant, blood preservative, etc., the aforementioned blood processing tool 1 is sterilized beforehand by means of moist heat sterilization (in an autoclave). It is preferable that the filter 51 be sterilized by gas sterilization (with ethylene oxide) or by radiation sterilization (with y rays). The sterilized filter is then built into the blood processing tool 1.

The aforementioned blood processing tool 1 is used in the following manner, wherein it is assumed that the filter 51 is used typically as a leukocyte-removing filter.
(1) In a first step, the blood collecting needle 41 of the blood collecting unit 2A is taken out, and the cap 44 is removed so that the needle tube 43 becomes exposed. The needle tube 43 is stuck into the donor's vessel to perform blood collection, wherein as much blood as necessary is collected in the first bag 3.

After blood collection has been completed, the needle tube 43 is covered again with the cap 44 and, if necessary, the tube 4 is sealed by fusion using a tube sealer and the sealed part is cut, whereupon the tube 4 connected to the blood collecting needle 41 is separated and removed.
(2) In a second step, the seal of the tube 5 is released (by means of a device that permits passage as the seal is broken) so that the blood inside the first bag 3 is discharged through the tube 5 and passes through the filter 51 for separation of leukocytes from the blood. To facilitate this procedure, the first blood collecting bag 3 is suspended at a given height on a stand so that the blood flows by gravity.

The blood that is discharged from the first bag 3 flows through the tube 5 and enters into the filter 51 through the inlet 52, so that leukocytes are removed (filtered out) by the filter medium. Leukocyte-free blood is discharged from the outlet 53 and introduced into the second bag 7 through the tube 6.
(3) After the leukocyte-free blood has been transferred to the second bag 7, the tube 6 (near the second bag 7) is sealed by fusion using a tube sealer, and the sealed portion thereof is cut, so that the blood collecting unit 2A and the filter 51 are separated from the set of second bags 7, 8, and 9.
(4) The second bags 7, 8, and 9 are placed together in the cup of a centrifuge. As the result of centrifugation, the leukocyte-free blood in the second bag 7 separates into two layers, the lower layer containing erythrocytes and the upper layer containing platelet-rich plasma. The pattern of separation of blood components depends on the conditions of centrifugation (such as rpm and duration).
(5) Next, the upper layer (containing platelet-rich plasma) in the second bag 7 is transferred to the second bag 8 in the following manner.

The second bag 7 is set on a bag pressing apparatus in order to separate and transfer the blood components. While the tube 13 is sealed with a clamp, the second bag 7 is gradually pressed, so that supernatant platelet-rich plasma is discharged from the second bag 7 and transferred into the second bag 8 through the tube 11, the branching connector 10, and the tube 12. Erythrocytes remain within the second bag 7.
(6) After the transfer of platelet-rich plasma has been completed, the clamp on the tube 13 is released. While the tube 12 is sealed with a clamp, the erythrocyte preserving solution contained in the second bag 9 is transferred to the second bag 7 (containing erythrocytes) through the tube 13, the branching connector 10, and the tube 11.
(7) The tube 11 is sealed midway by fusion bonding (using a tube sealer) and the sealed portion is cut in order to separate the second bag 7 from the second bags 8 and 9. The second bag 7 is shaken to thoroughly mix together the erythrocytes and the erythrocyte preserving solution.

At this point, the second bag 7 contains CRC (Concentrated Red Blood Cells).
(8) The second bags 8 and 9 are placed together into the cup of the centrifuge to perform centrifugation thereon.

As a result of centrifugation, the platelet rich plasma in the second bag 8 separates into platelets (precipitate) and platelet-poor plasma (supernatant).

The pattern of separation of the blood components depends on the conditions of centrifugation.
(9) The second bag 8 is set on a bag pressing apparatus in order to separate and transfer the blood components, and the second bag 8 is gradually pressed.

In this way, supernatant plasma is discharged from the second bag 8 and transferred into the second bag 9 through the tube 12, the branching connector 10, and the tube 13. This step should be carried out such that an adequate amount of plasma remains inside the second bag 8, which is necessary to prepare concentrated platelet plasma (platelet suspension).
(10) Then, the tubes 12 and 13 are sealed midway by fusion bonding (using a tube sealer), and the sealed portion is cut in order to separate the second bags 8 and 9 from each other, so that the platelet precipitate inside the second bag 8 is dispersed in the plasma.

Thus, the second bag 8, which contains platelets or platelet-concentrated (PC), and the second bag 9, which contains plasma or platelet-poor plasma (PPP), are obtained.

In this way, different kinds of blood products are obtained, including erythrocytes (concentrated red blood cells [CRC]) contained in the second bag 7, platelets (platelet concentrated [PC]) contained in the second bag 8, and plasma (platelet-poor plasma [PPP]) contained in the second bag 9.

The above-described procedure for performing blood separation and recovery is merely one example, and the present invention is not restricted in the kind of blood components that can be separated and recovered, the number and type of bags, or the order of steps.

The packaging material and the package for a medical instrument set (as shown in FIGS. 1 and 2) according to the present invention shall be described as follows.

The package 14 for a medical instrument set according to the present invention is composed of a packaging material 15, wherein the aforementioned blood processing tool 1 (or a medical instrument set) are housed therein. The blood processing tool 1 is composed of the first bag 3, the filter 51, the second bag 7, the second bag 8, and the second bag 9, which are sequentially placed on top of each other, with the first bag 3 being disposed closest to the opening 17.

The packaging material 15 is composed of a packaging material body 16 and a lid 20, the packaging material body 16 having an opening 17 through which the contents are taken out, wherein the lid 20 closes the opening 17.

The packaging material body 16 is a bag made of a flexible sheet or film. The raw material for the flexible sheet may be selected from polyolefins (such as polyethylene, polypropylene, polybutene, polybutadiene, ethylene-vinyl acetate copolymer (EVA), and ethylene-a-olefin copolymer), polyolefin blend resins, polyester (such as polyethylene terephthalate and polybutylene terephthalate), polyvinyl chloride, polyvinylidene chloride, vinyl chloride-vinylidene chloride copolymer, polyurethane, and combinations thereof (in the form of blended resins, polymer alloys, and laminates). The sheet material may also be formed of plastic, non-woven fabric, or paper (synthetic paper).

The sheet constituting the packaging material body 16 is not restricted to being a monolayer structure, but may also be formed as a laminate structure (composed of different materials), such as a coated film or a laminated film. The coated film may be composed of a single resin layer and a deposited coating layer of aluminum or an oxide (such as titanium oxide, aluminum oxide, and silicon oxide). The laminated film may be composed of a single resin layer and a metal foil (such as an aluminum film).

A laminate film offers the advantages of a constituent film. For example, the laminate film imparts improved impact resistance or anti-blocking properties to the packaging material body 16.

A sheet material with a metallic layer provides improved gas barrier properties. A laminate sheet with an aluminum foil provides improved gas barrier properties as well as providing a light blocking effect. A laminate sheet with a thin deposited layer of oxide (such as titanium oxide, aluminum oxide, and silicon oxide) provides improved gas barrier properties as well as good clarity (which makes the contents of the package visible).

The sheet constituting the packaging material body 16 is not particularly restricted in thickness, but should preferably have a thickness of 20 to 600 µm, or more preferably, a thickness of 30 to 300 µm.

The packaging material body 16 is formed by tightly sealing or bonding the periphery of the above-mentioned sheet. Sealing may be accomplished by heat sealing, highfrequency sealing, or ultrasonic sealing. Bonding may be accomplished using an adhesive or a solvent.

The method of sealing is not particularly restricted, and includes four-sided sealing (in which the periphery of the sheet is entirely sealed), three-sided sealing (in which a sheet is folded and three sides, excluding the fold, are sealed), two-sided sealing (in which both ends of a cylindrical sheet are sealed), and bag sealing (in which the opening of a blow-molded bag is sealed).

The sheet forming the packaging material body 16 may be produced by various methods, such as inflation, T-die extrusion, blow molding, dry lamination, hot-melt lamination, inflation coextrusion, T-die coextrusion, and hot pressing.

The packaging material body 16 has an opening 17 through which the medical instruments are taken out. The opening 17 has a specific shape, area and position, which are determined by the shape and size of the individual medical instruments, such as the first bag 3, the blood collecting needle 41, the filter 51, and the second bags 7 to 9.

The figure shows an elliptical opening 17, which is formed near the center of one side of the packaging material body 16. The opening may take on any other shape, such as a circle, a square, a rectangle, a trapezoid, and a rhombus. The position of the opening 17 is not restricted to the center of the packaging material body 16, but may also be formed near one end.

The opening 17 has a straight perforation 18 extending from the periphery thereof. The perforation 18 permits the sheet to be broken easily. After the lid has been peeled off, the opening 17 can be expanded by breaking the perforation 18. In other words, the perforation 18 serves to expand the opening 17.

The opening 17, which has been expanded by breaking the perforation, permits the medical instruments to be taken out easily from the packaging material 15.

The perforation 18 may be replaced by any other means that serves to expand the opening 17. This object may also be achieved by using a sheet having a linear or groove-like thin wall part.

The means for easy breakage is not restricted in position and pattern to those shown in the figure. For example, the perforation may be formed in a cross pattern, which radiates from the center of the opening 17.

When the packaging material 15 is not yet opened, the opening 17 remains closed by the lid 20. In this case, the perforation 18 is also covered by the lid 20. In this way, the packaging material 15 is protected from breathing through the perforation 18.

The lid 20 is formed from a sheet-like (or film-like) material, which is tightly bonded to the peripheral region (including the perforation 18) of the opening 17 of the packaging material body 16 by heat sealing, high-frequency sealing, or ultrasonic sealing, or through use of an adhesive or solvent. The lid 20 is peeled off partially or entirely (as shown in FIG. 2) in order to unseal the opening 17.

The sheet-like material constituting the lid 20 may have the same characteristic properties as the sheet-like material for the aforementioned packaging material body 16, and preferably should have the same gas barrier properties as the packaging material body 16.

The lid 20 includes a projection (or tab) 21 at one end thereof, which can be pinched by the fingers to facilitate peeling.

One medical instrument (or part thereof), which is to be used first among the blood processing tool 1, is held or fixed on the inside of the lid 20 (or on the inside of the packaging material 15). (The part of the medical instrument, which has been designated as being used first, need not necessarily be used first.) According to the embodiment of the present invention, an adhesive layer 22 (indicated by hatching in FIGS. 1 and 2) is formed on the inside (i.e., on a reverse side) of the lid 20, wherein the adhesive layer 22 holds the needle 41 (as the medical instrument to be used first) bonded thereto. Thus the needle 41 can be detached easily.

The adhesive layer 22 may be formed from any adhesive, such as acrylic, rubber, and silicone.

The packaging material according to the present invention is not limited to the one illustrated in the embodiment. The packaging material may be composed of a main body and a sheet-like lid, so that the packaging material is unsealed as the lid is peeled off. In this case, the main body is molded from a comparatively rigid resin and has a blister to hold the medical instrument set therein. The lid is fixed to the main body by heat sealing or by adhesive bonding.

Next, a description shall be given concerning a method of using the package 14 for the medical instrument set.

While the packaging material 15 is closed, the tab 21 of the lid 20 is pulled up, so that the lid 20 is mostly peeled off from the packaging material body 16, as shown in FIG. 1. Now, the opening 17 is exposed and unsealed. Simultaneously with the unsealing operation, the needle 41, which is held (fixed) on the inside of the lid 20, is taken out through the opening 17. In this way, the needle 41, which is to be used first, is taken out automatically from the packaging material 15 as the packaging material 15 is unsealed. This facilitates rapid and easy preparation and operation of blood collection.

Then, the needle 41 is peeled off from the adhesive layer 22 on the lid 20, and the cap 44 is removed so that the needle tube 43 is exposed. The needle tube 43 is stuck into the donor's vessel to perform blood collection. See the procedure (1) discussed above.

Alternatively, blood collection may be accomplished as shown in FIG. 2. In this case, although not illustrated, the first bag 3 is taken out through the opening 17, so that the blood collecting unit 2A is entirely removed from the packaging material 15. The first bag 3 may be taken out after the perforation has been entirely or partly broken and the opening 17 has been enlarged. This procedure permits the first bag 3 to be taken out easily and securely.

The next step is to take out the filter 51 and the second bags 7, 8, and 9 sequentially or all at once. Prior to this procedure, the perforation 18 is partially or entirely broken in order to enlarge the opening 17 and to facilitate removal of the second bags 7, 8 and 9.

The blood processing unit 2B, which has been taken out as described above, is used for blood collection according to the aforementioned steps (2) to (10).

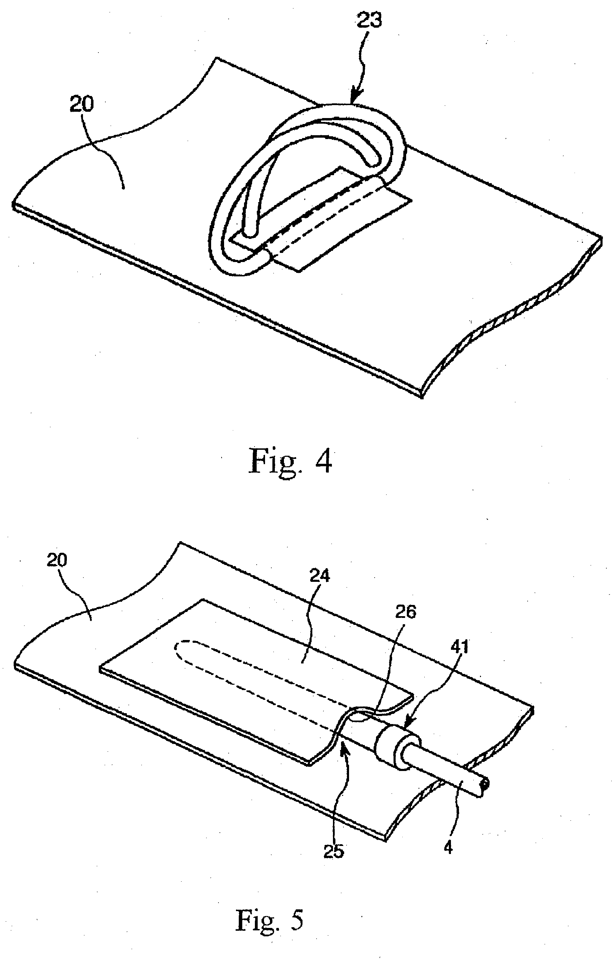

FIGS. 4 and 5 are perspective views of another embodiment of the means for fixing or holding the medical instrument (or part thereof) to be used first on the inside of the lid.

As shown in FIG. 4, the lid 20 has a string-like (or belt-like) holding member 23 capable of plastic deformation, which is attached to the inside thereof. The holding member 23 may be a resin-coated metal wire. The holding member 23 is wound around the needle 41 (which is the medical instrument to be used first), so that the needle 41 is held on and fixed to the lid 20.

The string-like holding member 23, which is capable of plastic deformation, permits the needle 41 to be held and fixed easily and securely, while also allowing easy removal of the needle 41. The holding member 23, which is a string-like body capable of plastic deformation, holds the medical instrument (not limited to the needle 41) securely regardless of its size and shape.

The aforementioned holding member may be replaced by any other members (metal or resin) having a string-like or belt-like form and which are capable of elastic deformation, which are molded into a shape suitable for holding the needle 41 (or the cap 44 or hub 42) fitted or inserted therein.

As shown in FIG. 5, a rectangular sheet 24 is provided on the inside of the lid 20, with three sides thereof (excluding one side 26 through which the medical instrument is inserted) being fixed by fusion-bonding or adhesion to form a pocket 25. The pocket 25 holds the needle 41, which is the medical instrument to be used first. The needle 41 is inserted into and taken out of the pocket 25 through a mouth 26.

The pocket 25 is formed with an adequate size and shape from a sheet 24 (having adequate stiffness, tensile strength, and surface friction), so that the sheet 24 prevents the needle 41 from falling out from the pocket 25 due to vibrations and shocks when the lid 20 is peeled off.

The holding means having the aforementioned pocket structure permits an operator to easily insert and take out the needle 41 from the pocket 25. The holding means can be produced easily and is suitable for mass production.

The mouth 26 of the pocket 25 should be positioned close to the tab 21 of the lid 20 (or close to the side of the lid 20 that is peeled off first). Such a structure permits an operator to easily take out the needle 41 from the pocket 25.

Incidentally, the aforementioned pocket 25 (with three sides sealed) may be modified as follows. The pocket 25 may have an open bottom (opposite to the mouth). In place of the sheet 24, the pocket 25 may be formed from a breathable mesh or fabric (woven fabric or non-woven fabric). Further, the pocket 25 may be formed from a plurality of belts. The pocket 25 may be constructed in any manner so long as it is capable of holding the medical instrument.

Next, an example of another means for holding or fixing the medical instrument (or part thereof) to be used first on the inside of the lid shall be described.

The example is identical in structure to that shown in FIGS. 1 and 2, except that the adhesive layer sheet 22 (indicated by hatching in FIGS. 1 and 2) is replaced by a magnet.

A sheet-like magnet may be formed from a resin or rubber compound along with a magnetic powder. The magnetic powder is not particularly restricted, so long as it has a comparatively strong magnetic attractive force. The magnetic powder may be, for example, an alloy of a transition metal (Fe, Ni, Co, etc.), a rare earth element (Nd, Pr, etc.), or boron.

The sheet-like magnet may be bonded to either the inside or the outside of the lid 20. Alternatively, the lid 20 itself may be formed from the sheet-like magnet.

If the needle tube 43 of the needle 41 is made of stainless steel or an iron alloy which is attracted magnetically, then the sheet-like magnet will be able to attract and hold the needle 41 on the inside of the lid 20 owing to its magnetic attractive force.

The structure in which the sheet-like magnet constitutes at least part of the lid is advantageous in that the needle 41 (which is the medical instrument to be used first) can be easily attached to and detached from the inside of the lid 20.

The present invention has been described above with reference to the illustrated embodiments. However, the present invention is not limited to the illustrated embodiments, but can be modified or incorporated with additional structures to produce the same effect. It is also possible to combine two or more of the features of the aforementioned embodiments.

The medical instrument set housed inside the packaging material is not limited to the aforementioned blood processing instruments. The medical instrument set may also include, for example, an extracorporeal circulation circuit (including an artificial lung), a blood dialyzing circuit (including a dialyzer), a catheter assembly, a catheter introducer, a guide wire assembly, an infusion set, and a medicine compounding kit.

### Industrial Applicability

According to the present invention, a medical instrument or part thereof, which is to be used first, is held or fixed on the inside of the lid of the packaging material body. The packaging material allows only the necessary medical instrument, from among a plurality of medical instruments making up a medical instrument set, to be taken out, with the remainder of the medical instruments being left inside the package in a previously established order, so that the medical instrument (or part thereof) to be used first appears as soon as the lid of the package is opened. Therefore, the packaging material is convenient for easy, quick, and accurate handling of a particular medical instrument selected from among the medical instruments making up the medical instrument set. Therefore, the present invention offers value to the industry.

## Claims

1. A packaging material (15) for housing a medical instrument set (2A, 2B) assembled from a plurality of medical instruments (41, 3, 51, 7, 8, 9), said packaging material comprising ;
a packaging material body (16) having an opening (17) therein through which the medical instrument (41, 3, 51, 7, 8, 9) is taken out; and
a lid (20) that closes said opening (17),
wherein said lid (20) is bonded or fusion-bonded to the periphery of said opening (17) of said packaging material body (16),
wherein said opening (17) is unsealed as said lid (20) is peeled off at least partly, **characterised in that** ;
said lid (20) holds or fastens, on an inside portion thereof, a medical instrument (41) or part thereof which is to be used first from among the medical instruments (41, 3, 51, 7, 8, 9) of said medical instrument set (2A, 2B),
wherein said medical instrument (41) to be used first is taken out through said opening (17) when said lid (20) is peeled off from said packaging material body (16).

2. The packaging material (15) as defined in claim 1,
wherein said packaging material (15) comprises means (18) for expanding said opening (17).

3. The packaging material (15) as defined in claim 2,
wherein said means (18) for expanding said opening (17) is formed on the periphery of said opening (17) so as to be broken easily.

4. The packaging material (15) as defined in claim 3,
wherein the means (18) for expanding said opening (17) comprises a perforation or a thin-walled part.

5. The packaging material (15) as defined in any of claims 2 to 4, wherein said means (18) for expanding said opening (17) is covered by said lid (20).

6. The packaging material (15) as defined in any of claims 1 to 5, wherein said medical instrument set (2A, 2B) comprises a plurality of medical instruments (41, 3, 51, 7, 8, 9) connected together by tubes (4, 5, 6, 11, 12, 13).

7. The packaging material (15) as defined in any of claims 1 to 6, wherein said medical instrument set (2A, 2B) includes at least one bag (3, 7, 8, 9) and one needle (41), and the medical instrument (41, 3, 51, 7, 8, 9) to be used first is said needle (41).

8. The packaging material (15) as defined in any of claims 1 to 6, wherein said medical instrument set (2A, 2B) comprises medical instruments (41, 3, 51, 7, 8, 9) including a first bag (3) for storing collected blood, a blood collecting needle (41) connected to said first bag (3) through a tube (4), a second bag (7) for storing blood or a blood component, and a filter (51) having an inlet (52), an outlet (53), and a filter medium for removing a specific component from blood introduced through said inlet (52), wherein said blood collecting needle (41) is the medical instrument to be used first.

9. The packaging material (15) as defined in any of claims 1 to 8, wherein said medical instrument (41) or part thereof to be used first is bonded to the inside of said lid (20).

10. The packaging material (15) as defined in any of claims 1 to 8, wherein said medical instrument (41) or part thereof to be used first is held on the inside of said lid (20) by means of a holding member (22, 23, 25) attached to the inside of said lid (20).

11. The packaging material (15) as defined in claim 10,
wherein said holding member (23) is a deformable string-like object.

12. The packaging material (15) as defined in any of claims 1 to 8, wherein an inside portion of said lid (20) includes a pocket (25), and wherein the medical instrument (41) or part thereof to be used first is inserted into said pocket (25).

13. The packaging material (15) as defined in any of claims 1 to 8, wherein said lid (20) comprises a sheet-like magnet disposed on at least a portion thereof, so that said medical instrument (41) or part thereof to be used first is magnetically held by said magnet.

14. A package housing medical instrument set (2A, 2B), which includes any of the packaging materials (15) defined in claims 1 to 13, and wherein the medical instrument set (2A, 2B) is housed inside the packaging material (15).

## Patentansprüche

1. Verpackungsmaterial (15) zur Unterbringung eines aus einer Vielzahl von medizinischen Instrumenten (41, 3, 51, 7, 8, 9) zusammengesetzten medizinischen Instrumentensets (2A, 2B), wobei das Verpackungsmaterial umfasst;
einen Verpackungsmaterialkörper (16) mit einer Öffnung (17) darin, durch welche das medizinische Instrument (41, 3, 51, 7, 8, 9) herausgenommen wird; und
einen Deckel (20), der die Öffnung (17) verschließt,
wobei der Deckel (20) an der Peripherie der Öffnung (17) des Verpackungsmaterialkörpers (16) gebunden oder schmelzgebunden ist,
wobei die Öffnung (17) unversiegelt ist, wenn der Deckel (20) zumindest teilweise abgelöst ist, **dadurch gekennzeichnet, dass**;
der Deckel (20) auf einen inneren Abschnitt davon ein medizinisches Instrument (41) oder Teil davon, welches unter den medizinischen Instrumenten (41, 3, 51, 7, 8, 9) des medizinischen Instrumentensets (2A, 2B) zuerst zu verwenden ist, hält oder befestigt,
wobei das zuerst zu verwendende medizinische Instrument (41) durch die Öffnung (17) entnommen wird, wenn der Deckel (20) von dem Verpackungsmaterialkörper (16) abgelöst wird.

2. Verpackungsmaterial (15) nach Anspruch 1,
wobei das Verpackungsmaterial (15) Mittel (18) zum Erweitern der Öffnung (17) umfasst.

3. Verpackungsmaterial (15) nach Anspruch 2,
wobei das Mittel (18) zum Erweitern der Öffnung (17) auf der Peripherie der Öffnung (17) gebildet ist, um leicht gebrochen zu werden.

4. Verpackungsmaterial (15) nach Anspruch 3,
wobei das Mittel (18) zum Erweitern der Öffnung (17) eine Perforierung oder einen dünnwandigen Teil umfasst.

5. Verpackungsmaterial (15) nach einem der Ansprüche 2 bis 4,
wobei das Mittel (18) zum Erweitern der Öffnung (17) durch den Deckel (20) abgedeckt ist.

6. Verpackungsmaterial (15) nach einem der Ansprüche 1 bis 5 ,
wobei das medizinische Instrumentenset (2A, 2B) eine Vielzahl von medizinischen Instrumenten (41, 3, 51, 7, 8, 9) umfasst, die durch Schläuche (4, 5, 6, 11, 12, 13) miteinander verbunden sind.

7. Verpackungsmaterial (15) nach einem der Ansprüche 1 bis 6,
wobei das medizinische Instrumentenset (2A, 2B) mindestens einen Beutel (3, 7, 8, 9) und eine Nadel (41) beinhaltet und das zuerst zu verwendende medizinische Instrument (41, 3, 51, 7, 8, 9) die Nadel (41) ist.

8. Verpackungsmaterial (15) nach einem der Ansprüche 1 bis 6,
wobei das medizinische Instrumentenset (2A, 2B) medizinische Instrumente (41, 3, 51, 7, 8, 9) umfasst beinhaltend eine erste Tasche (3) zum Speichern von gesammeltem Blut, eine durch einen Schlauch (4) mit dem ersten Beutel (3) verbundene Blutsammelnadel (41), einen zweiten Beutel (7) zum Speichern von Blut oder einer Blutkomponente, und einen Filter (51) mit einem Einlass (52), einem Auslass (53) und einem Filtermedium zum Entfernen einer spezifischen Komponente aus Blut, das durch den Einlass (52) eingeführt wird, wobei die Blutsammelnadel (41) das zuerst zu verwendende Instrument ist.

9. Verpackungsmaterial (15) nach einem der Ansprüche 1 bis 8,
wobei das medizinische Instrument (41) oder dessen Teil, der als erstes verwendet wird, an der Innenseite des Deckels (20) gebunden ist.

10. Verpackungsmaterial (15) nach einem der Ansprüche 1 bis 8,
wobei das zuerst zu verwendende medizinische Instrument oder Teil davon, der als erstes verwendet wird, auf der Innenseite des Deckels (20) mittels eines an die Innenseite des Deckels (20) angebrachten Halteelements (22, 23, 25) gehalten wird.

11. Verpackungsmaterial (15) nach Anspruch 10,
wobei das Halteelement (23) ein verformbarer schnurartiger Gegenstand ist.

12. Verpackungsmaterial (15) nach einem der Ansprüche 1 bis 8,
wobei ein innenseitiger Abschnitt des Deckels (20) eine Tasche (25) beinhaltet und wobei das zuerst zu verwendende medizinische Instrument (41) oder Teil davon in die Tasche (25) eingesetzt ist.

13. Verpackungsmaterial (15) nach einem der Ansprüche 1 bis 8,
wobei der Deckel (20) einen plattenartigen Magneten umfasst, der zumindest auf einen Abschnitt davon angeordnet ist, sodass das zuerst zu verwendende medizinische Instrument (41) oder Teil davon magnetisch durch den Magneten gehalten wird.

14. Verpackung, die ein medizinisches Instrumentenset (2A, 2B) unterbringt, welches Verpackungsmaterialien (15) nach einem der Ansprüche 1 bis 13 beinhaltet, und wobei das medizinische Instrumentenset (2A, 2B) innerhalb des Verpackungsmaterials (15) untergebracht ist.

## Revendications

1. Matériau d'emballage (15) pour loger un ensemble d'instruments médicaux (2A, 2B) assemblé à partir d'une pluralité d'instruments médicaux (41, 3, 51, 7, 8, 9), ledit matériau d'emballage comprenant :
un corps de matériau d'emballage (16) ayant une ouverture (17) à l'intérieur de ce dernier par laquelle on prend l'instrument médical (41, 3, 51, 7, 8, 9) ; et
un couvercle (20) qui ferme ladite ouverture (17),
dans lequel ledit couvercle (20) est relié ou relié par fusion à la périphérie de ladite ouverture (17) dudit corps de matériau d'emballage (16),
dans lequel ladite ouverture (17) n'est pas scellée étant donné que ledit couvercle (20) est détaché au moins partiellement, **caractérisé en ce que** :
ledit couvercle (20) maintient ou fixe, sur sa partie intérieure, un instrument médical (41) ou une partie de ce dernier qui doit être utilisé(e) en premier parmi les instruments médicaux (41, 3, 51, 7, 8, 9) dudit ensemble d'instruments médicaux (2A, 2B),
dans lequel ledit instrument médical (41) à utiliser en premier est pris par ladite ouverture (17) lorsque ledit couvercle (20) est détaché dudit corps de matériau d'emballage (16).

2. Matériau d'emballage (15) selon la revendication 1,
dans lequel ledit matériau d'emballage (15) comprend des moyens (18) pour expanser ladite ouverture (17).

3. Matériau d'emballage (15) selon la revendication 2,
dans lequel lesdits moyens (18) pour expanser ladite ouverture (17) sont formés sur la périphérie de ladite ouverture (17) afin d'être facilement cassés.

4. Matériau d'emballage (15) selon la revendication 3,
dans lequel les moyens (18) pour expanser ladite ouverture (17) comprennent une perforation ou une partie à paroi fine.

5. Matériau d'emballage (15) selon l'une quelconque des revendications 2 à 4, dans lequel lesdits moyens (18) pour expanser ladite ouverture (17) sont recouverts par ledit couvercle (20).

6. Matériau d'emballage (15) selon l'une quelconque des revendications 1 à 5, dans lequel ledit ensemble d'instruments médicaux (2A, 2B) comprend une pluralité d'instruments médicaux (41, 3, 51, 7, 8, 9) raccordés ensemble par des tubes (4, 5, 6, 11, 12, 13).

7. Matériau d'emballage (15) selon l'une quelconque des revendications 1 à 6, dans lequel ledit ensemble d'instruments médicaux (2A, 2B) comprend au moins un sachet (3, 7, 8, 9) et une aiguille (41), et l'instrument médical (41, 3, 51, 7, 8, 9) à utiliser en premier est ladite aiguille (41).

8. Matériau d'emballage (15) selon l'une quelconque des revendications 1 à 6, dans lequel ledit ensemble d'instruments médicaux (2A, 2B) comprend des instruments médicaux (41, 3, 51, 7, 8, 9) comprenant un premier sachet (3) pour stocker du sang collecté, une aiguille de prélèvement sanguin (41) raccordée audit premier sachet (3) par un tube (4), un second sachet (7) pour stocker du sang ou un composant sanguin, et un filtre (51) ayant une entrée (52), une sortie (53) et une couche filtrante pour retirer un composant spécifique du sang introduit par ladite entrée (52), dans lequel ladite aiguille de prélèvement sanguin (41) est l'instrument médical à utiliser en premier.

9. Matériau d'emballage (15) selon l'une quelconque des revendications 1 à 8, dans lequel ledit instrument médical (41) ou une partie de ce dernier à utiliser en premier est relié à l'intérieur dudit couvercle (20).

10. Matériau d'emballage (15) selon l'une quelconque des revendications 1 à 8, dans lequel ledit instrument médical (41) ou une partie de ce dernier à utiliser en premier, est maintenu sur l'intérieur dudit couvercle (20) au moyen d'un élément de maintien (22, 23, 25) fixé à l'intérieur dudit couvercle (20).

11. Matériau d'emballage (15) selon la revendication 10, dans lequel ledit élément de maintien (23) est un objet en forme de cordon déformable.

12. Matériau d'emballage (15) selon l'une quelconque des revendications 1 à 8, dans lequel une partie intérieure dudit couvercle (20) comprend une poche (25), et dans lequel l'instrument médical (41) ou une partie de ce dernier à utiliser en premier, est inséré(e) dans ladite poche (25).

13. Matériau d'emballage (15) selon l'une quelconque des revendications 1 à 8, dans lequel ledit couvercle (20) comprend un aimant en forme de feuille disposé sur au moins une partie de ce dernier, de sorte que ledit instrument médical (41) ou une partie de ce dernier à utiliser en premier est magnétiquement maintenu(e) par ledit aimant.

14. Emballage logeant l'ensemble d'instruments médicaux (2A, 2B), qui comprend l'un quelconque des matériaux d'emballage (15) selon les revendications 1 à 13, et dans lequel l'ensemble d'instruments médicaux (2A, 2B) est logé à l'intérieur du matériau d'emballage (15).
